# EUROPEAN PATENT APPLICATION

(11) **EP 4 570 923 A1**
(43) Date of publication of application: **18.06.2025**
(21) Application number: 24824385.9
(22) Date of filing: 02.04.2024
(51) Int. Cl.: C12Q 1/6886, C12Q 1/6858, C12N 15/11

(54) **KIT AND METHOD FOR DETECTING BLADDER CANCER**

(30) Priority: 13.10.2023 CN 202311328449
(71) Applicant: Qingdao Ruiside Medical Laboratory Co., Ltd., Qingdao, Shandong 266000 (CN)
(72) Inventor: CHEN, Mengmeng, Qingdao, Shandong 266000 (CN); ZHANG, Bingqiang, Qingdao, Shandong 266000 (CN); CHENG, Ying, Qingdao, Shandong 266000 (CN); YU, Junmei, Qingdao, Shandong 266000 (CN); LUAN, Yansong, Qingdao, Shandong 266000 (CN); ZHOU, Yang, Qingdao, Shandong 266000 (CN); LIU, He, Qingdao, Shandong 266000 (CN); SUN, Jing, Qingdao, Shandong 266000 (CN); HAN, Lihui, Qingdao, Shandong 266000 (CN); SUN, Yundong, Qingdao, Shandong 266000 (CN)
(74) Representative: Cabinet Chaillot
(86) International application number: PCT/CN2024/085465
(87) International publication number: WO 2025/077120

(57) **Abstract**

The present invention discloses a bladder cancer detection kit and detection method. The kit includes a DHFR PCR reaction solution, a Twist1 and TSC21 PCR reaction solution, a point mutation positive control, a methylation positive control and a negative control. According to the present invention, by detecting the point mutation of a DHFR gene and the methylation level of a promoter region of TSC21 and Twist1 genes in a sample, the joint detection of gene point mutation and methylation is achieved for determining whether the sample is cancerous. The kit is convenient and simple to operate, high in detection sensitivity and high in specificity, and has a very positive significance in the detection of bladder cancer.

## Description

### TECHNICAL FIELD

The present invention pertains to the technical field of biomedical testing, and in particular to a bladder cancer detection kit and a bladder cancer detection method.

### BACKGROUND

Bladder cancer is one of the most common malignant tumors in the urinary system, with about 80,000 new cases and about 33,000 deaths per year in China, both of which are in the 13^{th} place of malignant tumors. The morbidity rate and mortality rate of male are much higher than that of female, and the morbidity rate is the second highest among male urogenital malignant tumors. In recent years, the morbidity rate and mortality rate of bladder cancer in China are on the rise, and the prevention and control situation is not optimistic. There are about 550,000 new cases and 200,000 deaths worldwide every year. About 80% of patients with bladder cancer suffer from non-muscle-invasive bladder cancer (NMIBC). Although NMIBC is not life-threatening, 70% of patients will have tumor recurrence after surgery, and 15% of patients will have progression of staging and grading. Therefore, patients with bladder cancer usually require repeated reexaminations and bladder perfusion therapies after surgery, which are expensive.

*Chinese Guidelines for the Diagnosis and Treatment of Bladder Cancer* and Chinese Guidelines for the Diagnosis and Treatment of Urological and Andrological Diseases (2019 Edition) issued in 2021 by Chinese Urological Association (CUA), Chinese Urological Doctor Association (CUDA) and Chinese Anti-Cancer Association Genitourinary Oncology Committee (CACA-GU) indicate: DNA methylation is the most common way of epigenetic modification of tumors, and detection thereof has an important prospect in molecular diagnosis of tumors. It has been reported that the specific DNA methylation sites of bladder cancer in urine are detected by a PCR technology, and the sensitivity in low-grade and non-muscle-invasive bladder cancer is significantly better than urine exfoliative cytology and FISH, which shows a good diagnostic result and is expected to realize clinical transformation; and in particular, it has the significant advantages in the diagnosis of early, minimal, residual and recurrent tumors, and is expected to be used for early diagnosis and recurrence monitoring of bladder cancer, thereby reducing invasive cystoscopy and providing scientific basis for secondary electroresection.

The traditional examination method is not enough to meet the clinical requirements: the gold standard "cystoscopy + pathological biopsy" is an invasive examination method, which increases the pain of patients and leads to low compliance; and molecular diagnostic technologies such as urine cytology and FISH, which have been used in clinical practice for many years, are not sensitive and specific enough to meet the requirements of clinical diagnosis. However, through the gene methylation detection of urine exfoliated cells, non-invasive sampling is realized, the compliance of subjects receiving detection is improved, and unnecessary invasive examination is reduced, thereby generating a positive impact on the decision of the clinical diagnosis and treatment of bladder cancer and having high clinical value.

### SUMMARY

In view of the problems in the prior art, an objective of the present invention is to provide a bladder cancer detection kit and detection method. Whether a sample is cancerous is determined through the joint detection of point mutation of a DHFR gene and methylation of a Twist1 gene and a TSC21 gene of urine cells, so that a simple, convenient and accurate bladder cancer screening method is provided for clinical use.

According to the present invention, the NCBI database number of the DHFR gene sequence is NC_000005.10, and the NCBI database numbers of the Twist1 gene and the TSC21 gene are NC_000005.10 and NC_000002.12, respectively.

To achieve the objective of the present invention, the present invention provides the following technical solution: a bladder cancer detection kit comprising a DHFR PCR reaction solution, a Twist1 and TSC21 PCR reaction solution, a point mutation positive control, a methylation positive control and a negative control, where the DHFR PCR reaction solution is used for detecting whether point mutation occurs in a DHFR gene (RS1650697); the Twist1 and TSC21 PCR reaction solution is used for detecting the degree of methylation in at least one target region in a promoter region of a Twist1 gene and a TSC21 gene; the promoter region is not strictly limited to a region located in a promoter fragment, but a gene fragment in a region near the promoter; and the methylation detection regions of the Twist1 and TSC21 genes both include a plurality of CpGs.

The sequence of a to-be-detected region for the point mutation of the DHFR gene targeted by the DHFR PCR reaction solution is CTGCAGGTACTTTGTGAGTTTTGTCCTGCAGTTCCAGTTCCATGAAGCCCTGTGCA AGGAGGCAGGCTATGAGGGCCCACTGCACCAGTGTGACATCTACCGGTCCACCAA GGCAGGGGCCAAGCTCCGGTGTGTGGTGGGAAGCCGGGGGAAGTGGGAGGCAG AGAGGAGCGGCTGGCAAAGGGTGTGGCAGGAGGTGTC.

The sequence of a to-be-detected region for the methylation of the Twist1 gene targeted by the Twist1 and TSC21 PCR reaction solution is TTCGTTTTTTAGTTTTTTTTTTTCGTTTTATTTTTTTTTTTCGGGGTTTAATAATTCGTT TTTTTAAATTATTTAAAAACGATTTGGTTCGGGCGGTCGGTTTTTTTATTCGTTTTTT AGTCGTTTTTTTTTTTTTTTTTTCGTCGTTTTTTTTCGGCGGGCGCGGGGCGATTTTT TTTTTCGTCGGAGCGTGCGGGTAGCGTTTTCGAATTTTAGCGTAGTTTAGGAAGCG GTCGGAGGAGATTGTTTTGGTCGCGGTGGTAGTTTTATTCGGAGTGGTTGTGATAG TAGTAATGGTAA; and the sequence of a to-be-detected region for the methylation of the TSC2 gene targeted by the Twist1 and TSC21 PCR reaction solution is TTTGACAATTTTGAGCCAGGCTTTCAGGCAGCAGAGGCCTGGGTGGCAGTCTGTG GTGTGAAGTGGATTTAGTCACACACAGTCTGATGTTGTAAACCACATGGGCTCTAT TTACCTTCCTCTCCTAGGACTAGGAAGATTCTGTAGAGTATCGGAAAGCATTGGTTC ATGTGCTGCCAGGATTCATGTTGGCTCTGGTGAGCCAGGGACAGGGAACTGACCT GCAGGCAGCCCAAGCAGCCACCTACTCACCTCACAATGACAGGGACCCTCATCAT ACAGGTCAGGGCTCAACTCAAAGACAGGCGTGGCAGTGGGACA.

The DHFR PCR reaction solution includes DHFR forward and reverse primers, a DHFR probe, internal reference ACTB forward and reverse primers, an internal reference ACTB probe, a PCR buffer solution, MgCl₂, dNTP and a Taq enzyme; and the Twist1 and TSC21 PCR reaction includes Twist1 forward and reverse primers, a Twist1 probe, TSC21 forward and reverse primers, a TSC21 probe, internal reference ACTB forward and reverse primers, an internal reference ACTB probe, a PCR buffer solution, MgCl₂, dNTP and a Taq enzyme.

The Taq enzymes in the DHFR PCR reaction solution and the Twist1 and TSC21 PCR reaction solution of the present invention may be a Taq DNA polymerase commonly used in the art.

The formulation amounts of the PCR buffer solution, MgCl₂ and dNTP in the DHFR PCR reaction solution and Twist1 and TSC21 PCR reaction solution of the present invention can be selected according to conventional methods in the art, the appropriate formulation amounts of individual reagents can be selected and mixed, or the commercially available reaction system finished product can be directly selected, and the difference of these components has no significant impact on the experiment effect. The PCR buffer solution may be a conventional buffer in the art, for example, Tris of 30 mmol/L to 60 mmol/L and KCl of 10 mmol/L to 50 mmol/L, or Tris of 30 mmol/L to 600 mmol/L and KAc of 10 mmol/L to 50 mmol/L, or Tris of 30 mmol/L to 60 mmol/L and CsCl of 10 mmol/L to 50 mmol/L. These differences have no significant impact on the reagents and detection results described herein.

A base sequence of the DHFR forward primer is TGAGTTTTGTCCTGC, a base sequence of the DHFR reverse primer is ACACCGGAGCTTGGCCCCT, and a base sequence of the DHFR probe is AGGCTATGAGGGCCCACT; a base sequence of the Twist1 forward primer is TTTTTTTTCGGCGGGCGCGGGGCGA, a base sequence of the Twist1 reverse primer is CCGAATAAAACTACCACCGCGACC, and a base sequence of the Twist1 probe is TCGTCGGAGCGTGCGGGTAGC; a base sequence of the TSC21 forward primer is TTTGTAGAGTATCGGAAAGTATTGG, a base sequence of the TSC21 reverse primer is CTATATAATAAAAATCCCTATCA, and a base sequence of the TSC21 probe is TTTGTAGAGTATCGGAAAGTATTGG; and a base sequence of the internal reference ACTB forward primer is TAGGATTTTTATTTAG, a base sequence of the internal reference ACTB reverse primer is TGTGAATTTTTGTTAT, and a base sequence of the internal reference ACTB probe is TTTTAAGGGAGGAGT.

Two ends of the DHFR probe, the Twist1 probe, the TSC21 probe and the ACTB probe are respectively labeled with a fluorescent reporter group and a fluorescent quencher group; the fluorescent reporter group is selected from FAM, HEX, ROX, JOE, VIC, TET, NED, FITC, CY3 or CY5; and the fluorescent quencher group is selected from BHQ1, BHQ2, BHQ3, TAMRA, Eclipse and DABCYL.

In a specific embodiment of the present invention, the fluorescent reporter group at the 5' end of the DHFR fluorescent probe is FAM and the quencher group at the 3' end is BHQ1; the fluorescent reporter group at the 5' end of the internal reference gene fluorescent probe is VIC and the quencher group at the 3' end is BHQ1; the fluorescent reporter group at the 5' end of the Twist1 fluorescent probe is FAM and the quencher group at the 3' end is BHQ1; the fluorescent reporter group at the 5' end of the TSC21 fluorescent probe is VIC and the quencher group at the 3' end is BHQ1; and the fluorescent reporter group at the 5' end of the internal reference gene fluorescent probe is ROX and the quencher group at the 3' end is BHQ1.

The content of each component in the DHFR PCR reaction solution is: the DHFR forward primer of 0.2 µmol/L to 0.5 µmol/L, the DHFR reverse primer of 0.2 µmol/L to 0.5 µmol/L, the DHFR probe of 0.1 µmol/L to 0.5 µmol/L, the internal reference ACTB forward primer of 0.2 µmol/L to 0.5 µmol/L, the internal reference ACTB reverse primer of 0.2 µmol/L to 0.5 µmol/L, the internal reference ACTB probe of 0.1 µmol/L to 0.5 µmol/L, the PCR buffer solution, the MgCl₂ of 2 mmol/L to 8 mmol/L, the dNTP of 0.2 mmol/L to 0.4 mmol/L, and the Taq enzyme of 200 U/mL to 1000 U/mL.

The content of each component in the Twist1 and TSC21 PCR reaction solution is: the Twist1 forward primer of 0.2 µmol/L to 0.5 µmol/L, the Twist1 reverse primer of 0.2 µmol/L to 0.5 µmol/L, the Twist1 probe of 0.1 µmol/L to 0.5 µmol/L, the TSC21 forward primer of 0.2 µmol/L to 0.5 µmol/L, the TSC21 reverse primer of 0.2 µmol/L to 0.5 µmol/L, the TSC21 probe of 0.1 µmol/L to 0.5 µmol/L, the PCR buffer solution, the MgCl₂ of 2 mmol/L to 8mmol/L, the dNTP of 0.2 mmol/L to 0.4 mmol/L and the Taq enzyme of 200 U/mL to 1000 U/mL.

The methylation positive control is selected from DNA of a human bladder cancer cell 5637.

The point mutation positive control is selected from a DHFR positive synthetic plasmid.

The negative control is sterile water.

To achieve the objective of the present invention, the present invention further provides a bladder cancer detection method, including the following steps:
(1) extracting a detection sample DNA1 from a human urine sample;
(2) converting part of the detection sample DNA1 through bisulfite to obtain purified Bis-DNA;
(3) mixing the detection sample DNA1 with the DHFR PCR reaction solution in the kit of the present invention, using the detection sample DNA1 as a template, performing a PCR amplification reaction using a primer and probe combination in the DHFR PCR reaction solution, and detecting a target gene DHFR and the internal reference gene ACTB simultaneously according to multi-channel fluorescent probe labeling;
(4) mixing the purified Bis-DNA with the Twist1 and TSC21 PCR reaction solution in the kit of the present invention, using the purified Bis-DNA as a template, performing a PCR amplification reaction using a primer and probe combination in the Twist1 and TSC21 PCR reaction solution, and detecting the two target genes Twist1 and TSC21 and the internal reference gene ACTB simultaneously according to multi-channel fluorescent probe labeling; and
(5) determining whether the DHFR gene is mutated according to the Ct value of the DHFR gene detected in the step (3) as follows:
   determining that the DHFR gene is mutated if the Ct value of the DHFR gene is ≤ 32, and determining that the DHFR gene is not mutated if the Ct value of the DHFR gene is > 32 or there is no Ct value;
   determining the degree of methylation according to the ΔCt values (target Ct value - internal reference Ct value) of the Twist1 gene and the TSC21 gene detected in the step (4) as follows: substituting X1=ΔCt of Twist1 and X2=ΔCt of TSC21 into a regression equation Y=3.227-0.463*X1-0.059*X2, determining that the detection sample is positive for the methylation of the Twist1 gene and the TSC21 gene if Y≥0, and determining that the detection sample is negative for the methylation of the Twist1 gene and the TSC21 gene if Y<0; and
   interpreting the detection sample as positive for canceration when the point mutation occurs in the DHFR gene and/or the Twist1 and TSC21 genes are positive for methylation, and interpreting the detection sample as negative for canceration when the point mutation does not occur in the DHFR gene and the Twist1 and TSC21 genes are negative for methylation.

The present invention achieves the following advantages:
1. The present invention provides a bladder cancer detection kit, including a DHFR PCR reaction solution for detecting whether point mutation occurs in a DHFR gene (RS1650697), and a Twist1 and TSC21 PCR reaction solution for detecting the degree of methylation in at least one target region in a promoter region of a Twist1 gene and a TSC21 gene. The detection kit of the present invention detects three target genes DHFR, Twist1 and TSC21 in a detection sample. The DHFR gene, the Twist1 gene and the TSC21 gene are all closely related to the progression of bladder cancer. The DHFR gene detection includes mutation of an RS1650697 site, and the methylation detection regions of the Twist1 and TSC21 genes include a plurality of CpGs, which reduces the randomness of base changes compared with the detection of a single base.
2. ACTB is selected as an internal reference gene for the three target genes. The target genes and the internal reference genes are simultaneously detected according to the multi-channel fluorescent probe labeling. Finally, the experimental results are determined according to the Ct values of the three genes.
3. The present invention further provides a bladder cancer detection method, and provides a matching method for nucleic acid extraction from a sample and sulfite conversion. The DNA after nucleic acid extraction from the sample and DNA after sulfite conversion are used as templates for the PCR reaction, and the three target genes DHFR, Twist1 and TSC21 are detected simultaneously. According to the present invention, through the joint detection of DHFR point mutation, and Twist1 and TSC21 methylation, the differences which may be caused by site selection and a reaction system are effectively supplemented, and the accuracy of bladder cancer detection is improved.

### BRIEF DESCRIPTION OF THE DRAWINGS

FIG 1 is a schematic diagram of an overall structure of a kit according to the present invention; (reference numerals: 1. DHFR PCR reaction solution, 2. Twist1 and TSC21 PCR reaction solution, 3. methylation positive control, 4. point mutation positive control, and 5. point mutation negative control);
FIG 2 is a PCR amplification curve of DHFR, Twist1 and TSC21 genes detected in a typical bladder cancer urine cell sample according to the present invention;
FIG 3 is a PCR amplification curve of DHFR, Twist1 and TSC21 genes detected in a typical normal human urine cell sample according to the present invention;
FIG 4 shows the pathological and clinical diagnosis results of 40 urine cell samples; and
FIG 5 is a receiver operating characteristic (ROC) curve for the bladder cancer detection by a detection kit and a detection method according to the present invention and for the pathological and clinical diagnosis of bladder cancer.

### DESCRIPTION OF THE EMBODIMENTS

The present invention is further described in detail below with reference to the accompanying drawings and embodiments.

### Embodiment 1

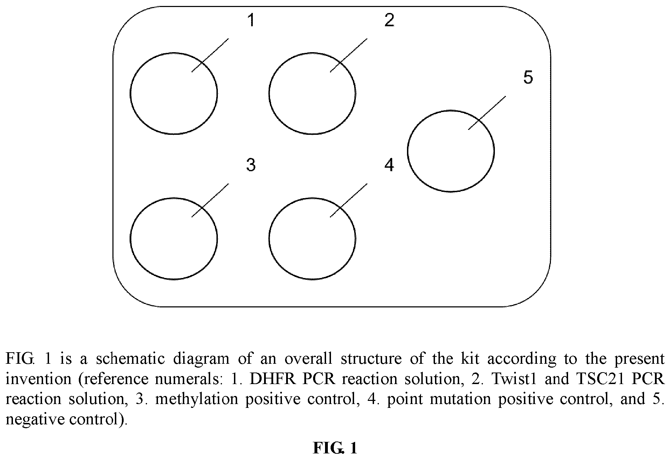

As shown in FIG 1, the present invention provides a bladder cancer detection kit, including a DHFR PCR reaction solution, a Twist1 and TSC21 PCR reaction solution, a point mutation positive control, a methylation positive control and a negative control, where the DHFR PCR reaction solution is used for detecting whether point mutation occurs in a DHFR gene (RS1650697); the Twist1 and TSC21 PCR reaction solution is used for detecting the degree of methylation in at least one target region in a promoter region of a Twist1 gene and a TSC21 gene; the promoter region is not strictly limited to a region located in a promoter fragment, but a gene fragment in a region near the promoter; and the methylation detection regions of the Twist1 and TSC21 genes both include a plurality of CpGs.

The nucleotide sequences of primer probes of the bladder cancer detection genes DHFR, Twist1 and TSC21 and the primer probe of the internal reference gene ACTB involved in this embodiment may be referenced to Table 1 below.

The sequence of a to-be-detected region for the point mutation of the DHFR gene targeted by the DHFR PCR reaction solution is CTGCAGGTACTTTGTGAGTTTTGTCCTGCAGTTCCAGTTCCATGAAGCCCTGTGCA AGGAGGCAGGCTATGAGGGCCCACTGCACCAGTGTGACATCTACCGGTCCACCAA GGCAGGGGCCAAGCTCCGGTGTGTGGTGGGAAGCCGGGGGAAGTGGGAGGCAG AGAGGAGCGGCTGGCAAAGGGTGTGGCAGGAGGTGTC.

The sequence of a to-be-detected region for the methylation of the Twist1 gene targeted by the Twist1 and TSC21 PCR reaction solution is TTCGTTTTTTAGTTTTTTTTTTTCGTTTTATTTTTTTTTTTCGGGGTTTAATAATTCGTT TTTTTAAATTATTTAAAAACGATTTGGTTCGGGCGGTCGGTTTTTTTATTCGTTTTTT AGTCGTTTTTTTTTTTTTTTTTTCGTCGTTTTTTTTCGGCGGGCGCGGGGCGATTTTT TTTTTCGTCGGAGCGTGCGGGTAGCGTTTTCGAATTTTAGCGTAGTTTAGGAAGCG GTCGGAGGAGATTGTTTTGGTCGCGGTGGTAGTTTTATTCGGAGTGGTTGTGATAG TAGTAATGGTAA; and the sequence of a to-be-detected region for the methylation of the TSC2 gene targeted by the Twist1 and TSC21 PCR reaction solution is TTTGACAATTTTGAGCCAGGCTTTCAGGCAGCAGAGGCCTGGGTGGCAGTCTGTG GTGTGAAGTGGATTTAGTCACACACAGTCTGATGTTGTAAACCACATGGGCTCTAT TTACCTTCCTCTCCTAGGACTAGGAAGATTCTGTAGAGTATCGGAAAGCATTGGTTC ATGTGCTGCCAGGATTCATGTTGGCTCTGGTGAGCCAGGGACAGGGAACTGACCT GCAGGCAGCCCAAGCAGCCACCTACTCACCTCACAATGACAGGGACCCTCATCAT ACAGGTCAGGGCTCAACTCAAAGACAGGCGTGGCAGTGGGACA.

The DHFR PCR reaction solution includes DHFR forward and reverse primers, a DHFR probe, internal reference ACTB forward and reverse primers, an internal reference ACTB probe, a PCR buffer solution, MgCl₂, dNTP and a Taq enzyme; and the Twist1 and TSC21 PCR reaction includes Twist1 forward and reverse primers, a Twist1 probe, TSC21 forward and reverse primers, a TSC21 probe, internal reference ACTB forward and reverse primers, an internal reference ACTB probe, a PCR buffer solution, MgCl₂, dNTP and a Taq enzyme.

The Taq enzymes in the DHFR PCR reaction solution and the Twist1 and TSC21 PCR reaction solution of the present invention may be a Taq DNA polymerase commonly used in the art.

The formulation amounts of the PCR buffer solution, MgCl₂ and dNTP in the DHFR PCR reaction solution and Twist1 and TSC21 PCR reaction solution of the present invention can be selected according to conventional methods in the art, the appropriate formulation amounts of individual reagents can be selected and mixed, or the commercially available reaction system finished product can be directly selected, and the difference of these components has no significant impact on the experiment effect. The PCR buffer solution may be a conventional buffer in the art, for example, Tris of 30 mmol/L to 60 mmol/L and KCl of 10 mmol/L to 50 mmol/L, or Tris of 30 mmol/L to 600 mmol/L and KAc of 10 mmol/L to 50 mmol/L, or Tris of 30 mmol/L to 60 mmol/L and CsCl of 10 mmol/L to 50 mmol/L. These differences have no significant impact on the reagents and detection results described herein.

Referring to Table 1, the base sequence of the DHFR forward primer is TGAGTTTTGTCCTGC (SEQ ID NO: 4), the base sequence of the DHFR reverse primer is ACACCGGAGCTTGGCCCCT (SEQ ID NO: 5), and the base sequence of the DHFR probe is AGGCTATGAGGGCCCACT (SEQ ID NO: 6); the base sequence of the Twist1 forward primer is TTTTTTTTCGGCGGGCGCGGGGCGA (SEQ ID NO: 7), the base sequence of the Twist1 reverse primer is CCGAATAAAACTACCACCGCGACC (SEQ ID NO: 8), and the base sequence of the Twist1 probe is TCGTCGGAGCGTGCGGGTAGC (SEQ ID NO: 9); the base sequence of the TSC21 forward primer is TTTGTAGAGTATCGGAAAGTATTGG (SEQ ID NO: 10), the base sequence of the TSC21 reverse primer is CTATATAATAAAAATCCCTATCA (SEQ ID NO: 11), and the base sequence of the TSC21 probe is TTTGTAGAGTATCGGAAAGTATTGG (SEQ ID NO: 12); and the base sequence of the internal reference ACTB forward primer is TAGGATTTTTATTTAG (SEQ ID NO: 13), the base sequence of the internal reference ACTB reverse primer is TGTGAATTTTTGTTAT (SEQ ID NO: 14), and the base sequence of the internal reference ACTB probe is TTTTAAGGGAGGAGT (SEQ ID NO: 15).

Two ends of the DHFR probe, the Twist1 probe, the TSC21 probe and the ACTB probe are respectively labeled with a fluorescent reporter group and a fluorescent quencher group; the fluorescent reporter group is selected from FAM, HEX, ROX, JOE, VIC, TET, NED, FITC, CY3 or CY5; and the fluorescent quencher group is selected from BHQ1, BHQ2, BHQ3, TAMRA, Eclipse and DABCYL.

In a specific embodiment of the present invention, the fluorescent reporter group at the 5' end of the DHFR fluorescent probe is FAM and the quencher group at the 3' end is BHQ1; the fluorescent reporter group at the 5' end of the internal reference gene fluorescent probe is VIC and the quencher group at the 3' end is BHQ1; the fluorescent reporter group at the 5' end of the Twist1 fluorescent probe is FAM and the quencher group at the 3' end is BHQ1; the fluorescent reporter group at the 5' end of the TSC21 fluorescent probe is VIC and the quencher group at the 3' end is BHQ1; and the fluorescent reporter group at the 5' end of the internal reference gene fluorescent probe is ROX and the quencher group at the 3' end is BHQ1.

The nucleotide sequences of primer probes of the bladder cancer detection genes DHFR, Twist1 and TSC21 and the primer probe of the internal reference gene ACTB involved in this embodiment may be referenced to Table 1 below.

**Table 1. Nucleotide Sequences of Gene Specific Primer Probe and Internal Reference Gene Primer Probe**

| Sequence Name | Oligonucleotide Sequence (5'-3') | Number |
|---|---|---|
| To-be-detected region of DHFR | CTGCAGGTACTTTGTGAGTTTTGTCCTGCAG TTCCAGTTCCATGAAGCCCTGTGCAAGGAG GCAGGCTATGAGGGCCCACTGCACCAGTGT GACATCTACCGGTCCACCAAGGCAGGGGCC AAGCTCCGGTGTGTGGTGGGAAGCCGGGGG AAGTGGGAGGCAGAGAGGAGCGGCTGGCA AAGGGTGTGGCAGGAGGTGTC | SEQ ID NO: 1 |
| To-be-detected region of Twist1 | TTCGTTTTTTAGTTTTTTTTTTTCGTTTTATTT TTTTTTTTCGGGGTTTAATAATTCGTTTTTTTA AATTATTTAAAAACGATTTGGTTCGGGCGGT CGGTTTTTTTATTCGTTTTTTAGTCGTTTTTTT TTTTTTTTTTTCGTCGTTTTTTTTCGGCGGGC GCGGGGCGATTTTTTTTTTCGTCGGAGCGTG CGGGTAGCGTTTTCGAATTTTAGCGTAGTTT AGGAAGCGGTCGGAGGAGATTGTTTTGGTC GCGGTGGTAGTTTTATTCGGAGTGGTTGTGA TAGTAGTAATGGTAA | SEQ ID NO: 2 |
| To-be-detected region of TSC21 | TTTGACAATTTTGAGCCAGGCTTTCAGGCAG CAGAGGCCTGGGTGGCAGTCTGTGGTGTGA AGTGGATTTAGTCACACACAGTCTGATGTTG TAAACCACATGGGCTCTATTTACCTTCCTCTC CTAGGACTAGGAAGATTCTGTAGAGTATCGG AAAGCATTGGTTCATGTGCTGCCAGGATTCA TGTTGGCTCTGGTGAGCCAGGGACAGGGAA CTGACCTGCAGGCAGCCCAAGCAGCCACCT ACTCACCTCACAATGACAGGGACCCTCATCA TACAGGTCAGGGCTCAACTCAAAGACAGGC GTGGCAGTGGGACA | SEQ ID NO: 3 |
| DHFR forward primer | TGAGTTTTGTCCTGC | SEQ ID NO: 4 |
| DHFR reverse primer | ACACCGGAGCTTGGCCCCT | SEQ ID NO: 5 |
| DHFR probe | AGGCTATGAGGGCCCACT | SEQ ID NO: 6 |
| Twist1 forward primer | TTTTTTTTCGGCGGGCGCGGGGCGA | SEQ ID NO: 7 |
| Twist1 reverse primer | CCGAATAAAACTACCACCGCGACC | SEQ ID NO: 8 |
| Twist1 probe | TCGTCGGAGCGTGCGGGTAGC | SEQ ID NO: 9 |
| TSC21 forward primer | TTTGTAGAGTATCGGAAAGTATTGG | SEQ ID NO: 10 |
| TSC21 reverse primer | CTATATAATAAAAATCCCTATCA | SEQ ID NO: 11 |
| TSC21 probe | TTTGTAGAGTATCGGAAAGTATTGG | SEQ ID NO: 12 |
| Internal reference ACTB forward primer | TAGGATTTTTATTTAG | SEQ ID NO: 13 |
| Internal reference | TGTGAATTTTTGTTAT | SEQ ID NO: 14 |
| ACTB reverse primer | | |
| Internal reference ACTB probe | TTTTAAGGGAGGAGT | SEQ ID NO: 15 |

In the kit of the present invention, the methylation positive control is selected from DNA of a human bladder cancer cell 5637; the point mutation positive control is selected from a DHFR positive synthetic plasmid; and the negative control is sterile water.

The specific components of the kit of the present invention are shown in Table 2 below:

**Table 2. Kit Component**

| Component | Main Component | Specification |
|---|---|---|
| DHFR PCR reaction solution | DHFR forward primer, DHFR reverse primer, DHFR probe, internal reference ACTB forward primer, internal reference ACTB reverse primer, internal reference ACTB probe, PCR buffer solution, dNTP, MgCl₂ and Taq enzyme. | 400 µL |
| Twist1 and TSC21 PCR reaction solution | Twist1 and TSC21 forward primers, Twist1 and TSC21 reverse primers, Twist1 and TSC21 probes, internal reference ACTB forward primer, internal reference ACTB reverse primer, internal reference ACTB probe, 10x buffer solution, PCR buffer solution, dNTP, MgCl₂ and Taq enzyme. | 400 µL |
| Methylation positive control | DNA of human bladder cancer cell 5637 | 50 µL |
| Point mutation positive control | DHFR positive synthetic plasmid | 50 µL |
| Negative control | Sterile water | 50 µL |

In the kit of the present invention, the content of each component in the DHFR PCR reaction solution is: DHFR forward primer of 0.2 µmol/L to 0.5 µmol/L, DHFR reverse primer of 0.2 µmol/L to 0.5 µmol/L, DHFR probe of 0.1 µmol/L to 0.5 µmol/L, internal reference ACTB forward primer of 0.2 µmol/L to 0.5 µmol/L, internal reference ACTB reverse primer of 0.2 µmol/L to 0.5 µmol/L, internal reference ACTB probe of 0.1 µmol/L to 0.5 µmol/L, PCR buffer solution, MgCl₂ of 2 mmol/L to 8 mmol/L, dNTP of 0.2 mmol/L to 0.4 mmol/L and Taq enzyme of 200 U/mL to 1000U/mL.

In the kit of the present invention, the content of each component in the Twist1 and TSC21 PCR reaction solution is: the Twist1 forward primer of 0.2 µmol/L to 0.5 µmol/L, the Twist1 reverse primer of 0.2 µmol/L to 0.5 µmol/L, the Twist1 probe of 0.1 µmol/L to 0.5 µmol/L, the TSC21 forward primer of 0.2 µmol/L to 0.5 µmol/L, the TSC21 reverse primer of 0.2 µmol/L to 0.5 µmol/L, the TSC21 probe of 0.1 µmol/L to 0.5 µmol/L, the PCR buffer solution, the MgCl₂ of 2 mmol/L to 8mmol/L, the dNTP of 0.2 mmol/L to 0.4 mmol/L and the Taq enzyme of 200 U/mL to 1000 U/mL.

### Embodiment 2

The present invention further provides a bladder cancer detection method, which comprises the following steps:
(1) The detection sample DNA1 was extracted: cell DNA1 was extracted from a human urine sample. After extraction, the concentration of the DNA and OD260/OD280 were measured using a Nano-500 micro-spectrophotometer, and the OD260/OD280 is between 1.6 and 2.0.
(2) Part of the detection sample DNA1 was converted with bisulfite to obtain purified Bis-DNA: after the extraction of the cell DNA, the extracted DNA was converted with bisulfite, unmethylated cytosine (C) was converted into uracil (U), methylated cytosine (C) remained unchanged, and the purified Bis-DNA was obtained. The obtained Bis-DNA should be detected timely or stored at -20°C for not more than 4 months for detection, or stored at -80°C for long-term storage.
(3) The detection sample DNA1 extracted in the step (1) was used as a template, and the primer and probe in the DHFR PCR reaction solution were combined for a PCR amplification reaction: for a DHFR target gene, ACTB was selected as an internal reference gene, and the target gene and the internal reference gene were detected simultaneously according to multi-channel fluorescent probe labeling.

The specific operation is as follows:
S3.1. preparation of reagents: the DHFR PCR reaction solution in the kit of the present invention and the detection sample DNA1;
S3.2. sample addition: 2 µL to 5 µL of detection sample DNA1, 15 µL of DHFR PCR reaction solution and sterile water with a volume up to 20 µL were added to each tube of eight-row tubes, with a final volume of 20 µL per tube, and each tube was tightly capped and centrifuged at a low speed instantaneously for detection on a PCR instrument; and
S3.3. PCR amplification: fluorescence detection channels were selected and amplification cycle parameters were set as shown in Table 3, note: ROX correction was not selected, and None was selected for quencher group, and after setup, a file was saved and a reaction program was run.

(4) A PCR amplification reaction was performed by using the purified Bis-DNA as a template and using a combination of the primers and the probes in the Twist1 and TSC21 PCR reaction solution: for the two target genes Twist1 and TSC21, ACTB was selected as an internal reference gene, and the target genes and the internal reference gene were detected simultaneously according to multi-channel fluorescent probe labeling.

The specific operation is as follows:
S4.1. preparation of reagents: the Twist1 and TSC21 PCR reaction solution in the kit of the present invention and the purified Bis-DNA template;
S4.2. sample addition: 2 µL to 5 µL of the purified Bis-DNA template, 15 µL of Twist1 and TSC21 PCR reaction solution and sterile water with a volume up to 20 µL were added to each tube of eight-row tubes, with a final volume of 20 µL per tube, and each tube was tightly capped and centrifuged at a low speed instantaneously for detection on a PCR instrument; and
S4.3. PCR amplification: fluorescence detection channels were selected and amplification cycle parameters were set as shown in Table 3, note: ROX correction was not selected, and None was selected for quencher group, and after setup, a file was saved and a reaction program was run.

(5) Whether the DHFR gene is mutated is determined according to the Ct value of the DHFR gene obtained in the step (3) as follows:
determining that the DHFR gene is mutated if the Ct value of the DHFR gene is ≤ 32, and determining that the DHFR gene is not mutated if the Ct value of the DHFR gene is > 32 or there is no Ct value;
determining the degree of methylation according to the ΔCt values (target Ct value - internal reference Ct value) of the Twist1 gene and the TSC21 gene detected in the step (4) as follows: substituting X1=ΔCt of Twist1 and X2=ΔCt of TSC21 into a regression equation Y=3.227-0.463*X1-0.059*X2, determining that the detection sample is positive for the methylation of the Twist1 gene and the TSC21 gene if Y≥0, and determining that the detection sample is negative for the methylation of the Twist1 gene and the TSC21 gene if Y<0; and
interpreting the detection sample as positive for canceration when the point mutation occurs in the DHFR gene and/or the Twist1 and TSC21 genes are positive for methylation, and interpreting the detection sample as negative for canceration when the point mutation does not occur in the DHFR gene and the Twist1 and TSC21 genes are negative for methylation.

**Table 3. Fluorescence Detection Channel Selection and Amplification Cycle Parameter Setting**

| Step | Temperature | Time | Number of Cycles |
|---|---|---|---|
| Step 1 (denaturation) | 95°C | 30 sec | 1 |
| Step 2 (amplification) | 95°C | 10 sec | 45 |
| Step 2 (amplification) | 60°C (fluorescence acquisition) | 30 sec | 45 |

Note: fluorescence acquisition is performed at 60°C in step 2 in Table 3, and the setting of the detection channel corresponds to the fluorescent reporter group set by the DHFR fluorescent probe, the Twist1 fluorescent probe, the TSC21 fluorescent probe and the internal reference gene fluorescent probe.

The logistic regression analysis of the present invention is as follows:
the detection results of three target genes DHFR, Twist1 and TSC21 of the normal sample and the bladder cancer sample were obtained through experiments, and a logistic regression equation for determining the gene methylation for Twist1 and TSC21 is: Y=A0-A1*X1- A2*X2, where A0, A1 and A2 are clinical coefficients, and the weight relationship of the clinical coefficients is as follows: Twist1 proportion coefficient A1=-0.463, TSC21 proportion coefficient A2=-0.059, and constant proportion coefficient A0=3.227.

The differences between the numbers of amplification cycle of the Twist1 and TSC21 genes and the internal reference gene ACTB are respective X1 and X2. X1=Ct value of Twist1 - Ct value of ACTB, and X2=Ct value of TSC21 - Ct value of ACTB were substituted into a regression equation Y=3.227-0.463*X1-0.059*X2 to determine the degree of methylation, it was determined that the to-be-detected sample was positive for the methylation of bladder cancer if Y≥0, and it was determined that the to-be-detected sample was negative for the methylation of bladder cancer if Y<0.

By jointly analyzing the methylation fitting regression of the Twist1 gene and the TSC21 gene and the DHFR point mutation to detect bladder cancer, the sensitivity and specificity are much higher than the detection analysis results of single gene such as DHFR, Twist1 and TSC21.

The accuracy test of the bladder cancer detection kit and detection method provided by the present invention on the bladder cancer detection is as follows:

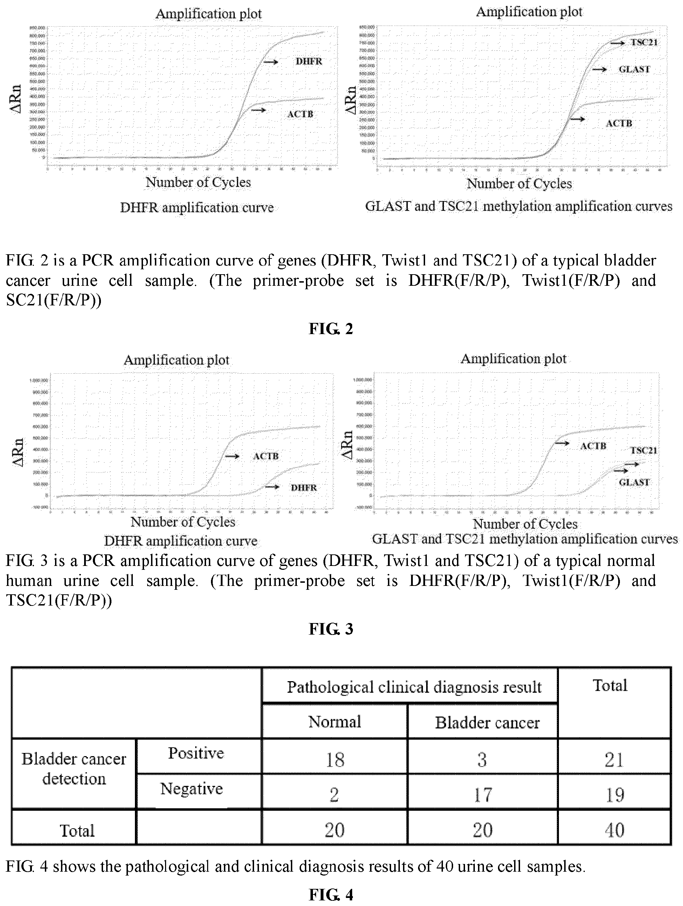

The results of the gene point mutation and methylation joint detection (DHFR, Twist1 and TSC21) on 40 urine cell samples are shown in FIG 2 and FIG 3. FIG 2 is a PCR amplification curve of DHFR, Twist1 and TSC21 genes detected in a typical bladder cancer urine cell sample according to the present invention. The primer-probe set in the DHFR PCR reaction solution and the Twist1 and TSC21 PCR reaction solution in the kit of the present invention is set to DHFR(F/R/P), Twist1(F/R/P) and TSC21(F/R/P). The result of detection on the typical bladder cancer urine cell sample by the kit of the present invention indicates that the Ct value of at least one of the DHFR gene, the Twist1 gene and the TSC21 gene in the bladder cancer samples is ≤32.

FIG 3 is a PCR amplification curve of DHFR, Twist1 and TSC21 genes detected in a typical normal human urine cell sample according to the present invention. The primer-probe set in the DHFR PCR reaction solution and the Twist1 and TSC21 PCR reaction solution in the kit of the present invention is set to DHFR(F/R/P), Twist1(F/R/P) and TSC21(F/R/P). The result of detection on the normal human urine cell samples by the kit of the present invention indicates that the Ct values of the DHFR gene, the Twist1 gene and the TSC21 gene in the normal human urine cell samples are all >32.

FIG 4 shows the pathological and clinical diagnosis results of 40 urine cell samples.

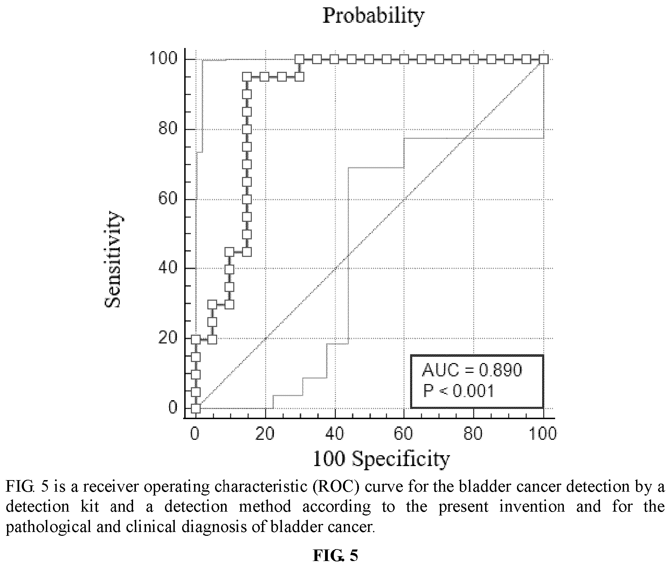

FIG 5 is a receiver operating characteristic curve for the bladder cancer detection by a detection kit and a detection method according to the present invention and for the pathological and clinical diagnosis of bladder cancer. FIG 5 indicates that detection by the detection kit of the present invention may obviously distinguish patients with bladder cancer and normal people. The area under the curve (AUC) of the ROC curve is 0.89 (95% CI 0.751-0.961; P<0.001).

The experimental data indicates that through comparison between the bladder cancer screening by the detection kit of the present invention and the clinical diagnosis result, the accuracy of the bladder cancer detection kit provided by the present invention can reach 87.5%, and the detection sensitivity and specificity are respectively 90% and 85%.

In conclusion, the bladder cancer detection kit disclosed by the present invention includes a DHFR PCR reaction solution, a Twist1 and TSC21 PCR reaction solution, a point mutation positive control, a methylation positive control and a negative control, can detect whether a point mutation occurs in an DHFR gene (RS1650697) and detect the degree of methylation in at least one target region in a promoter region of the Twist1 gene and the TSC21 gene, where the DHFR gene, the Twist1 gene and the TSC21 gene are all closely related to the progression of bladder cancer. By jointly detecting DHFR point mutation, and Twist1 and TSC21 methylation, the detection kit effectively supplements the differences which may be caused by site selection and reaction system, and improves the detection accuracy of bladder cancer. The detection method is easy and convenient to operate, has high detection sensitivity and good specificity, and has a very positive significance in the detection of bladder cancer.

## Claims

1. A bladder cancer detection kit, comprising a DHFR PCR reaction solution, a Twist1 and TSC21 PCR reaction solution, a point mutation positive control, a methylation positive control and a negative control, wherein the DHFR PCR reaction solution is used for detecting whether point mutation occurs in a DHFR gene (RS1650697); the Twist1 and TSC21 PCR reaction solution is used for detecting the degree of methylation in at least one target region in a promoter region of a Twist1 gene and a TSC21 gene; the promoter region is not strictly limited to a region located in a promoter fragment, but a gene fragment in a region near the promoter; and the methylation detection regions of the Twist1 and TSC21 genes both comprise a plurality of CpGs.

2. The bladder cancer detection kit according to claim 1, wherein the sequence of a to-be-detected region for the point mutation of the DHFR gene targeted by the DHFR PCR reaction solution is CTGCAGGTACTTTGTGAGTTTTGTCCTGCAGTTCCAGTTCCATGAAGCCCTGTGCA AGGAGGCAGGCTATGAGGGCCCACTGCACCAGTGTGACATCTACCGGTCCACCAA GGCAGGGGCCAAGCTCCGGTGTGTGGTGGGAAGCCGGGGGAAGTGGGAGGCAG AGAGGAGCGGCTGGCAAAGGGTGTGGCAGGAGGTGTC.

3. The bladder cancer detection kit according to claim 1, wherein the sequence of a to-be-detected region for the methylation of the Twist1 gene targeted by the Twist1 and TSC21 PCR reaction solution is TTCGTTTTTTAGTTTTTTTTTTTCGTTTTATTTTTTTTTTTCGGGGTTTAATAATTCGTT TTTTTAAATTATTTAAAAACGATTTGGTTCGGGCGGTCGGTTTTTTTATTCGTTTTTT AGTCGTTTTTTTTTTTTTTTTTTCGTCGTTTTTTTTCGGCGGGCGCGGGGCGATTTTT TTTTTCGTCGGAGCGTGCGGGTAGCGTTTTCGAATTTTAGCGTAGTTTAGGAAGCG GTCGGAGGAGATTGTTTTGGTCGCGGTGGTAGTTTTATTCGGAGTGGTTGTGATAG TAGTAATGGTAA; and the sequence of a to-be-detected region for the methylation of the TSC2 gene targeted by the Twist1 and TSC21 PCR reaction is TTTGACAATTTTGAGCCAGGCTTTCAGGCAGCAGAGGCCTGGGTGGCAGTCTGTG GTGTGAAGTGGATTTAGTCACACACAGTCTGATGTTGTAAACCACATGGGCTCTAT TTACCTTCCTCTCCTAGGACTAGGAAGATTCTGTAGAGTATCGGAAAGCATTGGTTC ATGTGCTGCCAGGATTCATGTTGGCTCTGGTGAGCCAGGGACAGGGAACTGACCT GCAGGCAGCCCAAGCAGCCACCTACTCACCTCACAATGACAGGGACCCTCATCAT ACAGGTCAGGGCTCAACTCAAAGACAGGCGTGGCAGTGGGACA.

4. The bladder cancer detection kit according to claim 1, wherein the DHFR PCR reaction solution comprises DHFR forward and reverse primers, a DHFR probe, internal reference ACTB forward and reverse primers, an internal reference ACTB probe, a PCR buffer solution, MgCl₂, dNTP and a Taq enzyme; and the Twist1 and TSC21 PCR reaction comprises Twist1 forward and reverse primers, a Twist1 probe, TSC21 forward and reverse primers, a TSC21 probe, internal reference ACTB forward and reverse primers, an internal reference ACTB probe, a PCR buffer solution, MgCl₂, dNTP and a Taq enzyme.

5. The bladder cancer detection kit according to claim 1, wherein a base sequence of the DHFR forward primer is TGAGTTTTGTCCTGC, a base sequence of the DHFR reverse primer is ACACCGGAGCTTGGCCCCT, and a base sequence of the DHFR probe is AGGCTATGAGGGCCCACT; a base sequence of the Twist1 forward primer is TTTTTTTTCGGCGGGCGCGGGGCGA, a base sequence of the Twist1 reverse primer is CCGAATAAAACTACCACCGCGACC, and a base sequence of the Twist1 probe is TCGTCGGAGCGTGCGGGTAGC; a base sequence of the TSC21 forward primer is TTTGTAGAGTATCGGAAAGTATTGG, a base sequence of the TSC21 reverse primer is CTATATAATAAAAATCCCTATCA, and a base sequence of the TSC21 probe is TTTGTAGAGTATCGGAAAGTATTGG; and a base sequence of the internal reference ACTB forward primer is TAGGATTTTTATTTAG, a base sequence of the internal reference ACTB reverse primer is TGTGAATTTTTGTTAT, and a base sequence of the internal reference ACTB probe is TTTTAAGGGAGGAGT.

6. The bladder cancer detection kit according to claim 4, wherein two ends of the DHFR probe, the Twist1 probe, the TSC21 probe and the ACTB probe are respectively labeled with a fluorescent reporter group and a fluorescent quencher group; the fluorescent reporter group is selected from FAM, HEX, ROX, JOE, VIC, TET, NED, FITC, CY3 or CY5; and the fluorescent quencher group is selected from BHQ1, BHQ2, BHQ3, TAMRA, Eclipse and DABCYL.

7. The bladder cancer detection kit according to claim 4, wherein
the content of each component in the DHFR PCR reaction solution is: the DHFR forward primer of 0.2 µmol/L to 0.5 µmol/L, the DHFR reverse primer of 0.2 µmol/L to 0.5 µmol/L, the DHFR probe of 0.1 µmol/L to 0.5 µmol/L, the internal reference ACTB forward primer of 0.2 µmol/L to 0.5 µmol/L, the internal reference ACTB reverse primer of 0.2 µmol/L to 0.5 µmol/L, the internal reference ACTB probe of 0.1 µmol/L to 0.5 µmol/L, the PCR buffer solution, the MgCl₂ of 2 mmol/L to 8 mmol/L, the dNTP of 0.2 mmol/L to 0.4 mmol/L, and the Taq enzyme of 200 U/mL to 1000 U/mL; and
the content of each component in the Twist1 and TSC21 PCR reaction solution is: the Twist1 forward primer of 0.2 µmol/L to 0.5 µmol/L, the Twist1 reverse primer of 0.2 µmol/L to 0.5 µmol/L, the Twist1 probe of 0.1 µmol/L to 0.5 µmol/L, the TSC21 forward primer of 0.2 µmol/L to 0.5 µmol/L, the TSC21 reverse primer of 0.2 µmol/L to 0.5 µmol/L, the TSC21 probe of 0.1 µmol/L to 0.5 µmol/L, the PCR buffer solution, the MgCl₂ of 2 mmol/L to 8mmol/L, the dNTP of 0.2 mmol/L to 0.4 mmol/L and the Taq enzyme of 200 U/mL to 1000 U/mL.

8. The bladder cancer detection kit according to claim 1, wherein the methylation positive control is selected from DNA of a human bladder cancer cell 5637; the point mutation positive control is selected from a DHFR positive synthetic plasmid; and the negative control is sterile water.

9. A bladder cancer detection method, comprising the following steps:
(1) extracting a detection sample DNA1 from a human urine sample;
(2) converting part of the detection sample DNA1 through bisulfite to obtain purified Bis-DNA;
(3) mixing the detection sample DNA1 with the DHFR PCR reaction solution in the kit according to claim 1, using the detection sample DNA1 as a template, performing a PCR amplification reaction using a primer and probe combination in the DHFR PCR reaction solution, and detecting a target gene DHFR and the internal reference gene ACTB simultaneously according to multi-channel fluorescent probe labeling;
(4) mixing the purified Bis-DNA with the Twist1 and TSC21 PCR reaction solution according to claim 1, using the purified Bis-DNA as a template, performing a PCR amplification reaction using a primer and probe combination in the Twist1 and TSC21 PCR reaction solution, and detecting the two target genes Twist1 and TSC21 and the internal reference gene ACTB simultaneously according to multi-channel fluorescent probe labeling; and
(5) determining whether the DHFR gene is mutated according to the Ct value of the DHFR gene detected in the step (3) as follows:
determining that the DHFR gene is mutated if the Ct value of the DHFR gene is ≤ 32, and determining that the DHFR gene is not mutated if the Ct value of the DHFR gene is > 32 or there is no Ct value;
determining the degree of methylation according to the ΔCt values (target Ct value - internal reference Ct value) of the Twist1 gene and the TSC21 gene detected in the step (4) as follows: substituting X1=ΔCt of Twist1 and X2=ΔCt of TSC21 into a regression equation Y=3.227-0.463*X1-0.059*X2, determining that the detection sample is positive for the methylation of the Twist1 gene and the TSC21 gene if Y≥0, and determining that the detection sample is negative for the methylation of the Twist1 gene and the TSC21 gene if Y<0; and
interpreting the detection sample as positive for canceration when the point mutation occurs in the DHFR gene and/or the Twist1 and TSC21 genes are positive for methylation, and interpreting the detection sample as negative for canceration when the point mutation does not occur in the DHFR gene and the Twist1 and TSC21 genes are negative for methylation.
